# EUROPEAN PATENT SPECIFICATION

(11) **EP 2 060 245 B1**
(45) Date of publication and mention of the grant of the patent: **29.08.2012**
(21) Application number: 08167045.7
(22) Date of filing: 20.10.2008
(51) Int. Cl.: A61F 5/08, A61L 9/04, A61L 15/58

(54) **Nasal dilator**
Nasaler Dilatator
Dilatateur nasal

(30) Priority: 13.11.2007 IT MI20072156
(43) Date of publication of application: 20.05.2009
(73) Proprietor: BIOFARMITALIA S.p.A., 20144 Milano (IT)
(72) Inventor: Pinna, Fausto, 20050, LESMO (MI) (IT); Pinna, Marco, 21051, ARCISATE (VA) (IT)
(74) Representative: Frignoli, Luigi

(56) References cited:
- EP-A- 1 679 085
- EP-A- 1 847 241
- WO-A-97/38651
- WO-A-98/25541
- WO-A-99/22678
- WO-A-99/27880
- WO-A-99/64102
- DE-A1- 10 251 636
- US-A- 3 655 129
- US-A- 5 769 089
- US-A- 5 842 469
- US-A1- 2002 000 227
- US-B1- 6 244 265
- US-B1- 6 390 089
- US-B1- 6 769 429

## Description

The present invention relates to a nasal dilator, in particular a nasal dilator of plastic material having a surface partially covered by a dermocompatible adhesive layer to enable it to be fixed to the nose of a user, to widen the nostrils and facilitate breathing.

Adhesive strips able to dilate the user's nostrils are known, to facilitate respiration; they consist of numerous mutually superposed components.

The documents WO9806360WO99/27880and US6244265describe a nasal strips comprising three components, namely at least one elastically deformable element, a strip of soft flexible material, and a dermocompatible adhesive substance applied to a surface of said soft strip.

The elastically deformable element (with a dilator function) consists particularly of at least one bar which is joined to the soft flexible strip by welding, or by sandwiching it within a pocket provided in the soft flexible strip.

These known structures result in numerous drawbacks.

In this respect when in use, after being made to adhere to the surface of the nose of a user of the strip, the elastically deformable element has to outwardly raise (away from the nose surface) the soft flexible strip which, via the adhesive substance , dilates the nose nostrils.

Hence in practice, as this very complex structure damps the force transmitted by the elastically deformable element to the soft strip for dilating the nostrils (especially if it has to outwardly raise the entire nose surface on which the soft material adheres), elastically deformable elements of high rigidity have to be used, with the result that the resistant force of the adhesive substance has to be increased, with consequent damage to the skin of the nose.

In this respect, as the adhesive substance is spread over the entire surface of the soft flexible strip, the traction exerted on this strip by the elastically deformable element is distributed over the entire strip surface in contact with the skin, so determining a reduction in the widening effect on the nostril by the nasal strip.

Finally, the use of three separate constituent components of the known nasal strip results in high costs.

The document WO 97/38651 discloses a nasal dilator consisting of a single elongated flexible element shaped as a beam exhibiting a rigidity increasing from its central thicker portion towards two attaching thin and flat shaped elements constituting extensions of said central portion, a skin compatible adhesive being applied to said flat shaped elements: such dilator is surely uncomfortable in use and especially no dermocompatible material could prevent its undesired quick detachment from the nose.

The technical aim of the present invention is therefore to provide a nasal dilator by which the stated technical drawbacks of the known art are eliminated.

Within the scope of this technical aim, an object of the invention is to provide a nasal dilator which can be applied directly (i.e. without interposing a strip of soft flexible material) to the surface of a user's nose to dilate the nostrils and facilitate respiration, without damaging the skin of the nose.

Another object of the invention is to provide a nasal dilator which can be applied to the skin very effectively, so enabling the force required to ensure dilation of the nostrils to be reduced (compared with that necessary with known nasal strips).

A further object of the invention is to provide a nasal dilator which is more economical than nasal strips of known type.

The technical aim, together with these and other objects, are attained according to the present invention by a nasal dilator characterised by consisting of an elongated elastically flexible element formed of plastic material and having a separate layer of dermocompatible adhesive material applied onto a first surface, characterized in that said element consists of a lamina having a thickness less than 400 micron and in that said separate layer of adhesive material is applied only at each of those two ends of the element most distant apart.

Preferably said lamina of plastic material has a thickness between 80 and 300 micron; each of said layers of adhesive material is without solvents and has a thickness less than 120 micron; and again preferably, said layers of adhesive material are permeable to vapour.

The aforesaid lamina of plastic material is preferably made of a material chosen from the group comprising polyester, PVC, polythene, polystyrene and nylon.

The dermocompatible adhesive material usable as a component of the nasal dilator is of known type, commonly used for adhesive plasters, i.e. consisting of a dermocompatible adhesive chosen from the group comprising water- or solvent-based acrylic adhesives, water or solvent based vinyl adhesives, polyurethane adhesives, resins of natural or synthetic origin, polyacrylates, natural polymers, gums, polyvinyl alcohols, cellulose, carrageens, and alginates.

Advantageously, as will be apparent from the examples of specific embodiments, the adhesive material, after its application to the support is water or solvent-free and is transpirable and permeable to vapour, just as it is taught in document EP 1 1679 805.

The nasal dilator of the invention can also be usefully covered (on the surface opposite that to which the dermocompatible adhesive material is applied) by a layer of vapour-permeable acrylic resin containing an aromatic substance or essential oil, such that these substances are slowly released when the dilator is applied to the nose of a user; slow release films of this type are disclosed in the U.S. 3,655,129.

The structure of the nasal dilator of the invention will be better understood from the description of a preferred embodiment thereof, with reference to the accompanying drawing, in which:
Figure 1 is a plan view of a nasal dilator according to the invention;
Figure 2 is a side view of the nasal dilator of Figure 1, ready to be applied to a user's nose;
Figure 3 shows the dilator of Figure 2 provided with a removable layer of siliconized paper protecting the adhesive material applied to the ends of a surface of the nasal dilator; and
Figure 4 is a side view of a second embodiment of a nasal dilator according to the invention provided, on a surface thereof opposite that comprising the adhesive material, with a layer of material containing an aromatic substance or essential oil;

The figures show a nasal dilator indicated overall by the reference numeral 1.

The nasal dilator 1 consists of an elastically yieldable elongated support lamina 2 having two separate adhesive layers on a surface 4 thereof.

Specifically, as shown in the Figures from 2 to 4, the nasal dilator presents two separate adhesive layers 7, 8 applied to the two opposing ends of the surface 4 of the lamina 2: these layers 7, 8 cover (when the dilator is in use) only a small portion of the nose surface, with consequent benefits in terms of tolerability and transpirability.

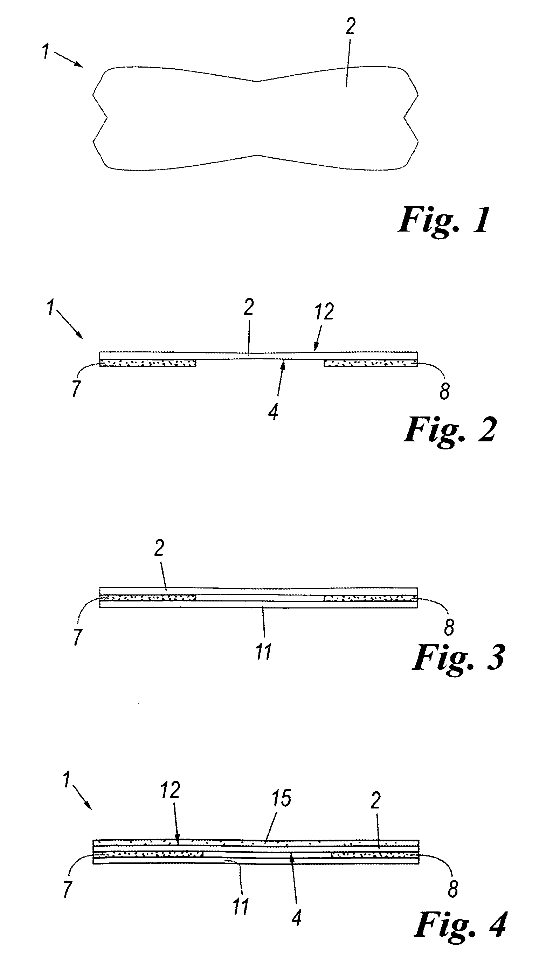

From Figure 3 it can be seen that a sheet 11 (for example of siliconized paper, as known in the art) is applied to the adhesive material layers 7, 8 and has to be removed before using the nasal dilator 1.

Figure 4 shows a more complex embodiment of the dilator, in which on that surface 12 of the lamina opposite the surface 4 on which the adhesive layers 7, 8 are applied, a layer 15 of a material (preferably an acrylic resin) containing an aromatic substance or essential oil is applied; this layer 15 is also free of solvents and is permeable to vapours (on termination of the application stage comprising heating in an oven, as described hereinafter). For use, a layer 11 protecting the adhesive 7, 8 is removed prior to use of the dilator, and the strip 1 is applied straddling the user's nose, with the adhesive layers 7, 8 pressed down and fixed in positions correspond with the nasal nostrils: the small size of the layers 7 and 8 leaves most of the surface of the nasal skin completely free.

The support 2 flexes when applied to the nose and tends, by virtue of its elasticity, to return to a straight configuration, and dilate the nostrils.

If present, the layer 15 releases the substances dispersed therein to gradually evolve aromatic and balsamic vapours, for example to facilitate respiration.

The following Examples describe the method of preparing nasal dilators with reference to the aforedescribed figures of the accompanying drawings.

### EXAMPLE 1

### Preparation of a nasal dilator comprising a polyester lamina of 100 micron thickness.

15.18 kg of solvent based acrylic adhesive (for example adhesive produced and sold under the name Durotak 2819 of National Starch & Chemical Co.) are fed cold into a container.

Using a patch spreading machine and with the aid of a compressed air pump the acrylic adhesive mixture is transferred onto the rotating cylinder doctor, having adjusted the doctor thickness to about 200 micron.

The doctor spreads the adhesive (to the set thickness) onto a continuous plastic web.

To form nasal dilators having only their ends provided with the adhesive layer, the adhesive is prevented from being spread over the entire surface of the plastic lamina by providing the doctor (in known manner) with dividers to form numerous doctor scrapers, each of which deposits on the plastic lamina a 40 mm band of adhesive, leaving 20 mm free of adhesive. Deposition continues for the entire length of the plastic web on which continuous adhesive bands of 40 mm width are hence present spaced apart by empty distances (i.e. without adhesive) of 20 mm.

The plastic web on which the adhesive is spread is formed of 100 micron thick polyester. After the adhesive has been spread in bands on this web, the web is passed at a speed of 8 metres/minute through four successive oven stations, the first oven station having its temperature controlled at 40ºC, the second at 50ºC, the third at 70ºC and the fourth at 80ºC.

At the oven exit the adhesive layers are completely free of solvents, which have evaporated in the oven stations; the thickness of the adhesive mass is about 41 g/m².

At the oven exit a strip of 80 g/m² siliconized paper is applied and pressed onto the surface of the polyester web where the adhesive material bands are provided, hence forming a combination which is rewound into a reel.

The result is that the adhesive layers, protected by the siliconized paper, bind to the polyester lamina.

The composite reel obtained is then cut into reels of lesser dimensions by cutting the 40 mm adhesive layers in half to obtain narrower reels of total width 60 mm, in which the side 20 mm portions are provided with an adhesive layer.

These reels are then cut and shaped by a punching machine as shown in Figure 1, to give nasal dilators protected by siliconized paper, which are then packaged.

A nasal dilator is shown in plan view in Figure 1 in which the numeral 1 indicates the dilator and the numeral 2 indicates the polyester lamina; in Figure 2 which shows the nasal dilator in side view, in which two separate portions 7 and respectively 8 of adhesive can be seen applied to the ends of the lamina 2; and in Figure 3 (similar to Figure 2) but in which the adhesive material 7, 8 is protected by a profiled sheet 11 of siliconized paper.

### EXAMPLE 2

### Preparation of a nasal dilator formed with a polyester lamina of 150 micron thickness.

30 kg of water based acrylic adhesive (methyl acrylate polymer - for example adhesive known by the registered name ACRONAL - 500 D adhesive) are fed cold into a container.

The preparation proceeds as indicated for example 1.

Using a patch spreading machine and with the aid of a compressed air pump the adhesive is transferred onto the rotating cylinder doctor, having adjusted the doctor thickness to about 200 micron; by means of the doctor the adhesive is applied to a web of plastic material.

To prevent the adhesive from being spread over the entire surface of the web, various doctor scrapers are formed, each of which deposits a band of 40 mm of adhesive, leaving 20 mm free of adhesive, and so on for the entire width of the polyester web having a thickness of 150 micron polyester.

The adhesive spread in bands on the polyester web passes through 4 oven stations, the first oven being controlled at 120ºC, the second at 130ºC, the third at 100ºC and the fourth at 90ºC, with a speed of 8 metres per minute.

At the oven exit the adhesive layers are completely free of solvents, which have evaporated in the oven stations; the thickness of the adhesive mass is about 50 g/m².

The polyester web is coupled to siliconized paper of 80 g/m² and rewound into a reel. The result is that the adhesive layers, protected and pressed by the siliconized paper, bind to the polyester web.

The reel obtained is then cut into reels of lesser dimensions by cutting the 40 mm adhesive layers in half to obtain reels of total width 60 mm, in which the side 20 mm portions are provided with an adhesive layer.

The rolled-up webs obtained in this manner are then cut and shaped by a punching machine to give nasal dilators (as shown in Figures from 1 to 3), which are then packaged.

### EXAMPLE 3

### Preparation of a nasal dilator formed with a PVC lamina of 200 micron thickness.

50 kg of solvent based acrylic adhesive (for example Duro-tak - 280A adhesive of National Starch & Chemical Co.) are fed cold into a container. The preparation proceeds as indicated in Example 1, but adjusting the doctor thickness to about 360 micron and spreading the adhesive on a PVC web of 200 micron thickness and then passing the adhesive spread on PVC through four oven stations, the first being controlled at 60ºC, the second at 70ºC, the third at 80ºC and the fourth at 90ºC, with a speed of 8 metres per minute.

At the oven exit the adhesive is completely free of solvents, which have evaporated in the oven stations; the thickness of the adhesive mass is about 50 g/m².

The PVC is then coupled to siliconized paper of 80 g/m² and rewound into a reel. The result is that the adhesive layers, pressed and protected by the siliconized paper, bind to the PVC.

The reel obtained in this manner is then cut into reels of lesser width by cutting the 40 mm adhesive layers in half to obtain reels of total width 60 mm, in which the side 20 mm portions are provided with an adhesive layer.

Finally, the rolled-up adhesive strips are shaped by a punching machine as shown in Figures from 1 to 3, and are then packaged.

### EXAMPLE 4

### Preparation of a nasal dilator of balsamic action formed with a polyester lamina of 100 micron thickness.

15.18 kg of solvent based acrylic adhesive (for example Duro-tak - 2819 adhesive of National Starch & Chemical Co.) are fed cold into a container.

The preparation proceeds as already indicated in example 1.

Using an adhesive plaster spreading machine and with the aid of a compressed air pump the mixture is fed onto the rotating cylinder doctor, having adjusted the doctor thickness to about 200 micron. In the same manner as described in Example 1, separate continuous bands of adhesive are deposited on a surface of a continuous polyester web of thickness 100 micron; this web is then passed through four oven stations, in the same manner and with the same characteristics as already described.

At the oven exit the polyester web is press-coupled to 80 g/m² siliconized paper and rewound into a reel. In this manner a composite web is obtained in which the adhesive bands, protected by the siliconized paper, grip the surface of the polyester web which is then rewound to form a reel.

Finally, on the other surface of the polyester web (that on which the adhesive bands are not present, a mixture is spread prepared in the following manner.

20 kg of a binder consisting of an aqueous dispersion of an acrylic and vinyl ester-based polymer (for example Binder 9011 produced and marketed by the company ICMA) are fed into an agitator, heated to 30ºC and mixed very slowly. 1.5 kg of eucalyptol essential oils and 0.5 kg of mint essential oil are fed in. These are mixed together for 10 minutes and then left standing for 30 minutes.

Using a transfer pump, this mixture is transferred onto a doctor blade set at 300 micron and spread over said other surface of the polyester web on that surface of the polyester web free of adhesive and not covered by the siliconized paper.

The composite web obtained in this manner is then passed through three separate successive oven stations, the first oven being controlled at 120ºC, the second at 110ºC, the third at 100ºC, with a speed of 6 metres per minute. At the oven exit this mixture layer containing essential oils is completely free of water, which has evaporated in the oven stations, while the essential oils remain trapped in the binder; the thickness of the mass is about 100 g/m².

At the oven exit the polyester web, coupled on one side to the siliconized paper and covered on the other side with the aromatic resin, is rewound into a reel.

This reel is then cut into reels of lesser dimensions by cutting the 40 mm adhesive layers in half to obtain reels of total width 60 mm, in which the side 20 mm portions are provided with an adhesive layer.

The composite web obtained in this manner is then unwound from the reel and made to pass through a punching machine, where profiled nasal dilators are cut out (as shown in Figure ) and are then immediately packaged. Each nasal dilator obtained in Example 4 comprises a profiled polyester lamina 2, on one surface 4 of which (and at the two ends of the lamina) are applied two separate portions 7 and respectively 8 of adhesive protected by a shaped sheet 11 of siliconized paper, while on the other surface (indicated by the numeral 112) of the other surface of the polyester lamina 2 a layer 15 of the mixture containing essential oils is applied.

Each nasal dilator, when applied to a person's nose, simultaneously performs two activities: it dilates the nostrils while slowly releasing balsamic aromas useful for example in the case of a cold.

## Claims

1. A nasal dilator (1) consisting of an elongated elastically flexible element (2) formed of plastic material and having a separate layer (7,8) of dermocompatible adhesive material applied onto a first surface (4) thereof at each of those two ends of the element (2) most distant apart, wherein said element consists of a lamina, **characterised in that** said (2) lamina (2) has a thickness less than 400 microns.

2. A nasal dilator as claimed in claim 1, **characterised in that** said lamina (2) of plastic material has a thickness between 80 and 300 micron.

3. A nasal dilator as claimed in claims 1 or 2, **characterised in that** each of said layers (7,8) of adhesive material is without solvents, has a thickness less than 120 micron and is permeable to vapour.

## Patentansprüche

1. Nasen-Dilatator, welcher aus einem länglichen elastischen flexiblen Element (2) besteht, welches aus einem Kunststoffmaterial ausgebildet ist und eine separate Schicht (7, 8) eines hautverträglichen klebenden Materials aufweist, die auf eine erste Oberfläche (4) davon an denjenigen zwei Enden des Elements (2), welche am weitesten entfernt sind, aufgetragen ist, wobei das Element aus einem Blättchen besteht, wobei das Blättchen (2) eine Dicke von weniger als 400 Mikrometer aufweist.

2. Nasen-Dilatator nach Anspruch 1, **dadurch gekennzeichnet, dass** das Blättchen (2) eines Kunststoffmaterials eine Dicke zwischen 80 und 300 Mikrometer aufweist.

3. Nasen-Dilatator nach Anspruch 1 oder 2, **dadurch gekennzeichnet, dass** jede der Schichten (7, 8) eines klebenden Materials kein Lösungsmittel ist, eine Dicke von weniger als 120 Mikrometer aufweist und für Dampf durchlässig ist.

## Revendications

1. Dilatateur nasal (1), constitué d'un élément allongé flexible de façon élastique (2) formé en matière plastique et ayant une couche séparée (7, 8) de matière adhésive dermo-compatible appliquée sur une première surface (4) de celui-ci à chacune des deux extrémités de l'élément (2) les plus éloignées l'une de l'autre, dans lequel ledit élément est constitué d'une lame,
**caractérisé en ce que** ladite lame (2) présente une épaisseur inférieure à 400 microns.

2. Dilatateur nasal selon la revendication 1, **caractérisé en ce que** ladite lame (2) en matière plastique présente une épaisseur comprise entre 80 et 300 microns.

3. Dilatateur nasal selon les revendications 1 ou 2, **caractérisé en ce que** chacune desdites couches (7, 8) de matière adhésive est sans solvant, présente une épaisseur inférieure à 120 microns et est perméable à la vapeur.
